(19) Europäisches Patentamt
European Patent Office
Office européen des brevets

(11) Numéro de publication : **0 231 139 B1**

(12) # FASCICULE DE BREVET EUROPEEN

(45) Date de publication du fascicule du brevet : **04.12.91 Bulletin 91/49**

(51) Int. Cl.$^5$ : **C07D 405/12,** C07D 307/79, C07D 311/58, A61K 31/335, A61K 31/40

(21) Numéro de dépôt : **87400194.4**

(22) Date de dépôt : **29.01.87**

(54) **Nouveaux dérivés de dihydrobenzofuranne - et de chromane -carboxamides, leurs procédés de préparation et leur utilisation comme neuroleptiques.**

Le dossier contient des informations techniques présentées postérieurement au dépôt de la demande et ne figurant pas dans le présent fascicule.

(30) Priorité : **30.01.86 FR 8601279**

(43) Date de publication de la demande : **05.08.87 Bulletin 87/32**

(45) Mention de la délivrance du brevet : **04.12.91 Bulletin 91/49**

(84) Etats contractants désignés : **AT BE CH DE ES FR GB GR IT LI LU NL SE**

(56) Documents cités :
**EP-A- 0 124 783**
**EP-A- 0 147 044**
**WO-A-84/03281**

(73) Titulaire : **Laboratoires DELAGRANGE**
**1, Avenue Pierre-Brossolette**
**F-91380 Chilly-Mazarin (FR)**

(72) Inventeur : **Franceschini, Jacqueline**
**28, Avenue Larroumes**
**F-94240 L'Hay-les-Roses (FR)**
Inventeur : **Margarit, Josette**
**69, avenue de Suffren**
**F-75007 Paris (FR)**

EP 0 231 139 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux dérivés de dihydrobenzofuranne- et de chromane-carboxamide de formule (I) :

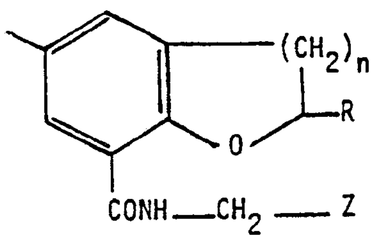

(I)

dans laquelle:

– R représente un atome d'hydrogène ou un groupe méthyle
– n est égal à 1 ou 2
– Z est un groupe de formule :

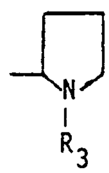

dans laquelle $R_3$ représente un groupe cycloalkylalkyle ou cycloalcénylalkyle,
– Y est un atome de chlore ou un groupe cycloalkylméthylsulfonyle,

ainsi que leur sels d'addition d'acides pharmacologiquement acceptables et leurs isomères optiques.

L'invention concerne également les composés suivants :

a) le N-(1-cyclohexénylméthyl 2-pyrrolidinylméthyl) 5-méthylsulfamoyl 2-méthyl 2,3-dihydrobenzofuranne 7-carboxamide,
b) le N-(1-cyclohexénylméthyl 2-pyrrolidinylméthyl) 6-méthylsulfamoyl chromane 8-carboxamide,
c) le N-(1-cyclopropylméthyl 2-pyrrolidinylméthyl) 6-éthylsulfonyl chromane 8-carboxamide,
d) le N-(1-cyclopropylméthyl 2-pyrrolidinylméthyl) 5-éthylsulfonyl 2-méthyl 2,3-dihydrobenzofuranne 7-carboxamide et
e) le N-(1-cyclohexénylméthyl 2-pyrrolidinylméthyl) 6-éthylsulfonyl chromane 8-carboxamide,

ainsi que leur sels d'addition d'acides pharmacologiquement acceptables et leurs isomères optiques.

D'après le brevet EP-0147044, on connaît des dérivés de dihydrobenzofuranne - carboxamide de formule analogue à la formule (I) des composés de l'invention, où n et R peuvent avoir les mêmes valeurs que dans la présente invention, où Y peut être un atome d'halogène et Z un groupe 2-pyrrolidinyle substitué, en particulier un groupe 1-alkyl 2-pyrrolidinyle.

D'après le brevet EP-0124783, on connaît des dérivés de dihydrobenzofuranne - et de chromane - carboxamide de formule analogue à la formule (I) dos composés de l'invention, où n et R peuvent avoir les mêmes valeurs que dans la présente invention, où Y peut âtre un groupe alkylsulfamoyle ou alkylsulfonyle et Z un groupe 2-pyrrolidinyle substitué, en particulier un groupe 1-alkyl 2-pyrrolidinyle.

les composés de ces deux brevets sont décrits comme des agents antipsychotiques.

les composés de la présente invention ont une structure très proche de celle des composés de l'art connu, les valeurs de R, n et Y pouvant être identiques et le groupe Z étant un groupe 2-pyrrolidinyle substitué. Ces composés diffèrent cependant des composés de l'invention au moins par le substituant de la pyrrolidine.

On a trouvé que les composés selon l'invention étaient doués d'une activité neuroleptique particulièrement importante, susceptible d'application dans le traitement des psychoses.

L'invention concerne également la préparation des composés de formule (I) et des composés (a), (b), (c), (d) et (e). les composés de formule (I) peuvent être préparés par réaction d'un acide de formule (II) :

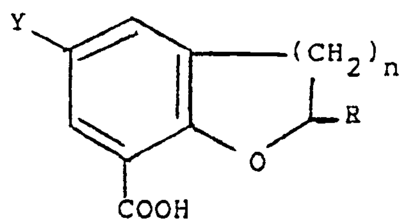

(II)

dans laquelle R, Y et n sont définis comme précédemment, ou de l'un de ses dérivés réactifs, avec une amine de formule (III) :

$H_2N—CH_2—Z$ (III)

dans laquelle Z est défini comme précédemment.

Ils peuvent également être préparés par réaction d'un acide de formule (II) telle que définie ci-dessus, ou l'un de ses dérivés réactifs, avec une dihaloalkylamine de formule (IV) :

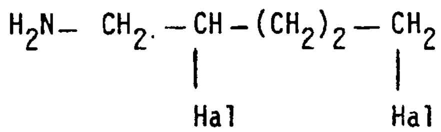

(IV)

dans laquelle Hal représente un atome d'halogène,
puis réaction du composé obtenu, de formule (V) :

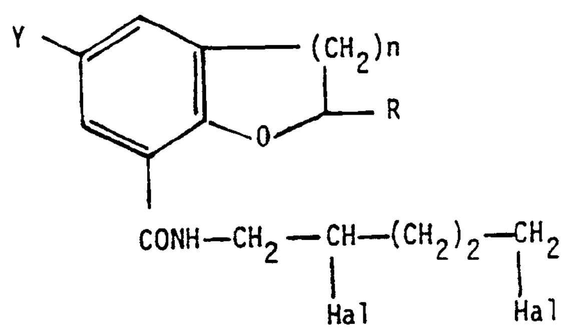

(V)

dans laquelle R, n et Hal sont définis comme précédemment, avec une amine de formule (VI) :

$H_2N—R_3$ (VI)

dans laquelle $R_3$ est défini comme précédemment.

les composés (a), (b), (c), (d) et (e) peuvent être préparés de façon analogue, par réaction de l'acide convenablement substitué correspondant, ou de l'un de ses dérivés réactifs, avec la 1-cyclohexénylméthyl 2-aminométhyl pyrrolidine ou la 1-cyclopropylméthyl 2-aminométhyl pyrrolidine, ou par réaction de l'acide correspondant, ou de l'un de ses dérivés réactifs, avec une dihalo-alkylamine de formule (IV) puis réaction du composé obtenu avec la cyclohexénylméthylamine ou la cyclopropylméthylamine.

Les dérivés réactifs de l'acide de formule (II) qui sont utilisés dans le procédé de l'invention comprennent, en particulier, les halogènures d'acide, les esters, les anhydrides symétriques et les anhydrides mixtes.

Le dérivé réactif de l'acide de formule (II) peut être utilisé dans la réaction d'amidification, directement après sa préparation ou après avoir été isolé du milieu réactionnel.

La réaction d'amidification peut être réalisée en présence d'un solvant tel que l'acétone, la méthyléthylcétone, le chloroforme et le diméthylformamide.

Les composés de l'invention peuvent être isolés sous forme de bases ou transformés en sels d'addition d'acide par réaction avec un acide minéral ou organique.

Les isomères optiques des composés de formule (I) peuvent être préparés par combinaison chimique des composés racémiques correspondants, avec un acide optiquement actif ou par réaction d'un acide de formule (II) avec un isomère optique de l'amine de formule (III), ou dans le cas où R est un groupe méthyle, par réaction d'une amine de formule (III) avec un isomère optique de l'acide de formule (II).

Les exemples suivants illustrent la préparation des composés de l'invention :

Exemple 1 : N - (1-cyclohexénylméthyl 2-pyrrolidinylméthyl) - 5-méthylsulfamoyl 2-méthyl-2,3-dihydrobenzofuranne 7-carboxamide.

-Acide 5-chlorosulfonyl 2-méthyl 2,3-dihydrobenzofuranne 7-carboxylique.

Dans un ballon de 1 litre, on a introduit 630 g de chlorhydrine sulfurique. On a refroidi à 0°C puis ajouté par portions, 160 g d'acide 2-méthyl 2,3-dihydrobenzofuranne 7-carboxylique finement pulvérisés, en maintenant la température entre 0° et 5°C par refroidissement dans un bain de glace.
Le mélange a été agité 1 heure à 5°C et 1 heure à la température ambiante puis versé sur de la glace en agitant et en maintenant la température à 0°C par refroidissement dans un bain glacé et introduction de glace dans le ballon. Les cristaux formés ont été essorés, lavés à l'eau et séchés à l'air.
Poids obtenu : 213 g (Rdt = 85,5%).

-Acide 5-méthylsulfamoyl 2-méthyl 2,3-dihydrobenzofuranne 7-carboxylique.

Dans un ballon de 2 litres, on a introduit 108 g d'une solution aqueuse de méthylamine à 40% et 108 ml d'eau. On a refroidi à 5°C puis ajouté 193 g d'acide 5-chlorosulfonyl 2-méthyl 2,3-dihydrobenzofuranne 7-carboxylique par portions de 10 g en refroidissant dans un bain de glace et sel de manière à maintenir la température à 5°C. Après chaque addition d'acide on a ajouté 14,5 ml d'une solution de 140 ml de lessive de soude à 30% dans 140 ml d'eau.
La solution obtenue a été diluée, filtrée puis acidifiée avec de l'acide chlorhydrique concentré jusqu'à virage du rouge congo.
Le précipité obtenu a été essoré, lavé et séché à 50°. Après recristallisation dans le méthanol, on a obtenu 119 g d'acide (P.F. = 214°C. rdt = 63%).

-Chlorure de 5-méthylsulfamoyl 2-méthyl 2,3-dihydrobenzofuranne 7-carbonyle.

Dans un ballon de 1 litre, on a introduit 232 g de chlorure de thionyle et 66 g d'acide 5-méthylsulfamoyl 2-méthyl 2,3-dihydrobenzofuranne 7-carboxylique. On a chauffé au bain d'eau jusqu'à dissolution puis rajouté 66 g d'acide et chauffé à nouveau jusqu'à dissolution. L'excès de chlorure de thionyle a été éliminé par distillation sous vide jusqu'à poids constant puis le résidu a été traité à l'éther de pétrole.
le produit pâteux obtenu a été essoré et séché sous vide.

- N-(1-cyclohexénylméthyl 2-pyrrolidinylméthyl)-5-méthylsulfamoyl 2-méthyl 2,3-dihydrobenzofuranne 7-carboxamide.

Dans un ballon de 2 litres, on a introduit 95 g de 1-cyclohexénylméthyl 2-aminométhyl pyrrolidine et 380 ml de chloroforme. On a refroidi à 5°C puis versé goutte à goutte une solution de 142 g de chlorure de 5-méthylsulfamoyl 2-méthyl 2,3-dihydrobenzofuranne 7-carbonyle dans 300ml de chloroforme, en maintenant la température entre 5° et 10°C par refroidissement extérieur.
On a laissé ensuite remonter la température puis repris le milieu réactionnel à l'eau. Après élimination du chloroforme, la solution restante a été filtrée et alcalinisée par de l'amoniaque à 20% jusqu'à virage de la phénolphtaléine.
Le précipité, solidifié après addition d'éther, a été essoré, lavé à l'eau et séché à 50°C.

Les 170 g de base obtenus ont été dissous à chaud dans 510 ml d'éthanol absolu. La solution bouillante a été filtrée puis les cristaux formés en refroidissant ont été essorés, lavés à l'éthanol et séchés à 50°C.
On a obtenu 101 g de base qui ont été recristallisés dans 300 ml d'éthanol absolu.
Poids obtenu : 86 g (P.F. = 155 - 156°C rdt = 39%).

Exemple 2 : N-(1-cyclohexénylméthyl 2-pyrrolidinylméthyl ) 6-méthylsulfamoyl chromane 8-carboxamide.

- Acide 6-chlorosulfonyl chromane 8-carboxilique.

Dans un ballon de 1 litre, on a introduit 930 ml de chlorhydrine sulfurique puis ajouté peu à peu, 165 g d'acide 8-chromane carboxylique, en maintenant la température entre 0°C et 10°C par refroidissement. La solution a été agitée 1 heure à 10°C, puis laissée à la température ambiante pendant une nuit. Elle a ensuite été versée peu à peu sur de la glace, en agitant et maintenant la température à 0°C en refroidissant extérieurement et en introduisant de la glace dans le ballon.

Les cristaux formés ont été essorés, lavés à l'eau et séchés à l'air.
Poids obtenu = 254 g (rdt = 99%).

- Acide 6-méthylsulfamoyl chromane 8-carboxylique

Dans un ballon de 1 litre, on a introduit 207 g d'une solution aqueuse de méthylamine à 40% puis, par portions, 123 g d'acide 6-chlorosulfonyl chromane 8-carboxylique finement pulvérisés, en maintenant la température entre 0° et 5°C, par refroidissement.
Le mélange a été maintenu à 5°C pendant 45 minutes puis on a laissé remonter la température. La solution obtenue a été diluée avec 1 litre d'eau, filtrée puis acidifiée par 125 ml d'acide chlorhydrique concentré. L'acide obtenu a été essoré, lavé à l'eau et séché à 50°C.
Poids obtenu = 107 g (P.F. = 204°C rdt = 90%).

- N-(1-cyclohexénylméthyl 2-pyrrolidinylméthyl) - 6-méthylsulfamoyl chromane 8-carboxamide.

Dans un ballon de 1 litre, on a introduit 76 g d'acide 6-méthylsulfamoyl chromane 8-carboxylique, 200 ml de chloroforme et 28,5 g de triéthylamine. On a refroidi à 0°C puis ajouté goutte à goutte, 30,5 g de chloroformiate d'éthyle en maintenant la température entre 0° et 5°C, par refroidissement dans un bain glacé. On a agité le mélange à cette température pendant 30 minutes puis ajouté goutte à goutte, entre 0° et 5°C, une solution de 55,5 g de 1-cyclohexénylméthyl 2-aminométhyl pyrrolidine dans 50 ml de chloroforme.
On a ensuite laissé remonter la température puis abandonné le mélange réactionnel pendant une nuit. La solution obtenue a été reprise à l'eau puis le chloroforme a été distillé. Les cristaux de chlorhydrate ont été redissous à chaud après addition de 1,8 litre d'eau. La solution bouillante a été filtrée puis alcalinisée par addition de lessive de soude à 30% jusqu'à virage de la phénolphtaléine. L'huile qui s'est formée a été refroidie, décantée et extraite au chlorure de méthylène. On a séché la solution chlorométhylènique sur carbonate de potassium puis distillé de chlorure de méthylène en terminant sous vide, jusqu'à poids constant.
Les 126 g de produit obtenus ont été redissous à chaud dans 250 ml d'éthanol absolu. Les cristaux formés par refroidissement ont été essorés, lavés à l'éthanol et séchés. On a obtenu 99,5 g de produit qui ont été recristallisés dans 200 ml d'éthanol.
Poids obtenu : 90,5 g (P.F. = 144,5°C - 145,5°C - rdt = 72%)

Exemple 3 : Méthane sulfonate de N-(1-cyclohexénylméthyl 2-pyrrolidinylméthyl) 5-cyclopropylméthylsulfonyl 2-méthyl 2,3-dihydrobenzofuranne 7-carboxamide.

- Acide 5-mercapto 2-méthyl 2,3-dihydrobenzofuranne 7-carboxylique.

Dans un ballon de 6 litres, on a introduit 216 g d'acide 5-chlorosulfonyl 2-méthyl 2,3-dihydrobenzofuranne 7-carboxylique, 585 ml d'acide acétique et 348 g d'étain, puis on a ajouté goutte à goutte 1560 ml d'acide chlorhydrique concentré (d= 1,18) en maintenant la température entre 45° et 55°C. On a chauffé ensuite pendant 2 heures à 55°- 60°C jusqu'à dissolution totale de l'étain, puis filtrée la solution qui a été versée dans 12 litres d'eau. Les cristaux formés ont été essorés, lavés à l'eau et séchés à 40°.
Poids obtenu : 130,5 g (P.F. = 158°C - P.M.= 225 - rdt = 79,5%).

- Acide 5-cyclopropylméthylthio 2-méthyl 2,3-dihydrobenzofuranne 7-carboxylique.

Dans un ballon de 2 litres, on a dissous 73 g de potasse dans 84 ml d'eau, puis ajouté 387 ml d'éthanol absolu et 130,5 g d'acide 5-mercapto 2-méthyl 2,3-dihydrobenzofuranne 7-carboxilique. On a versé ensuite rapidement 86 g de bromure de cyclopropylméthyle puis chauffé au reflux pendant 1 heure. Après addition d'eau, on a filtré puis acidifié la solution par addition d'acide chlorhydrique concentré jusqu'à virage,du' rouge congo. Le précipité formé a été essoré, lavé à l'eau et séché à 40°C.
Poids obtenu = 133 g (PF = 90°C - rdt = 87%)

- Acide 5-cyclopropylméthylaulfonyl 2-méthyl 2,3-dihydrobenzofuranne 7-carboxilique.

Dans un ballon de 2 litres, on a dissous 133 g d'acide 5-cyclopropylméthylthio 2-méthyl 2,3-dihydrobenzofuranne 7-carboxilique dans 735 ml d'acide acétique puis ajouté 315 ml d'eau oxygénée à 107,5 volumes.
La température a est élevée de 20° à 40°C.
On a ensuite chauffé au reflux pendant 5 heures, puis distillé la majeure partie de l'acide acétique et repris le résidu par 3 litres d'eau.
Les cristaux formés ont été essorés, lavés à l'eau et séchés à 40°C. Poids obtenu : 133,5 g (PF = 178°C - rdt

= 89%).

- N- (1-cyclohexénylméthyl 2-pyrrolidinylméthyl) 5-cyclopropylméthylsulfonyl 2-méthyl 2,3-dihydrobenzofuranne 7-carboxamide.

Dans un ballon de 1 litre, on a introduit 97 g d'acide 5-cyclopropylméthylsulfonyl 2-méthyl 2,3-dihydrobenzofuranne 7-carboxylique, 350 ml de chloroforme et 34 g de triéthylamine. On a refroidi la solution à 0°C puis ajouté goutte à goutte, 36 g de chloroformiate d'éthyle, en maintenant la température entre 0° et 5°C. On a agité pendant 1 heure entre 0° et 5°C puis versé goutte à goutte, entre 5°C et 10°C, 67 g de 1-cyclohexénylméthyl 2-aminométhyl pyrrolidine. On a agité le mélange pendant 1 heure, entre 5° et 10°C puis laissé remonter la température. La solution obtenue a été reprise par 2 litres d'eau. On a ajouté de l'acide acétique jusqu'à ph = 4 puis distillé le chloroforme. La solution restante a été filtrée puis alcalinisée par de la lessive de soude à 30% jusqu'à virage de la phénolphtaléine. L'huile formée a été décantée et extraite à l'éther puis la solution éthérée a été séchée sur carbonate de potassium. L'éther a été distillé en terminant sous vide jusqu'à poids constant.
Poids obtenu = 146 g (rdt = 94%).

- Méthane sulfonate de N- (1-cyclohexénylméthyl 2-pyrrolidinylméthyl) 5-cyclopropylméthylsulfonyl 2-méthyl 2,3-dihydrobenzofuranne 7-carboxamide.

On a dissous 141 g de N(1-cyclohexénylméthyl 2-pyrrolidinylméthyl) 5-cyclopropylméthylsulfonyl 2-méthyl 2,3-dihydrobenzofuranne 7-carboxamide dans 340 ml d'acétate d'éthyle puis ajouté 29 g d'acide méthane sulfonique.
Les cristaux formés ont été essorés, lavés à l'acétate d'éthyle et séchés à 40°C.
On a obtenu 133 g de produit qui ont été redissous à chaud dans 400 ml d'alcool isopropylique.
La solution bouillante a été filtrée avec du noir. Les cristaux formés en refroidissant ont été essorés, lavés à l'alcool isopropylique et séchés à 40°C.
Le produit obtenu a été abandonné à l'air jusqu'à ce que son poids soit devenu stable. On a obtenu 120 g d'un produit contenant une mole d'eau (PF = 126°C - rdt = 68%).

Exemple 4 : N-(1-cyclopropylméthyl 2-pyrrolidinylméthyl) 6-cyclopropylméthylsulfonyl chromane 8-carboxamide

- Acide 6-mercapto chromane 8-carboxylique.

Dans un ballon de 6 litres, on a introduit 238 g d' acide 6-chlorosulfonyl chromane 8-carboxylique et 645 ml d'acide acétique. On a chauffé le mélange à 80°C et ajouté 384 g d'étain puis refroidi à 50°C. On a ensuite versé goutte à goutte, 1720 ml d'acide chlorhydrique (d = 1,18) en maintenant la température entre 55° et 60°C d'abord par refroidissement dans un bain glacé puis par chauffage au bain d'eau. On a continué de chauffer ensuite à 60° pendant 3 heures puis on a versé la solution dans 6 litres d'eau. Le précipité formé a été essoré, lavé avec 1 litre d'acide chlorhydrique dilué puis à l'eau et séché à l'air.
Poids obtenu : 168 g (PF = 130° - 133°C - rdt = 93%).

- Acide 6-cyclopropylméthylthio chromane 8-carboxylique.

Dans un ballon de 3 litres, on a dissous 90 g de potasse dans 90 ml d'eau puis ajouté 900 ml d'éthanol et 137 g d'acide 6- mercapto chromane 8- carboxylique. On a ensuite versé goutte à goutte, 162 g de tosylate de cyclopropylméthyle à 90%.
On a chauffé au reflux pendant 3 heures puis distillé une partie de l'alcool et repris le résidu à l'eau.
La solution obtenue a été filtrée avec du noir puis acidifiée par de l'acide chlorhydrique concentré.
Les cristaux formés ont été essorés, lavés à l'eau et séchés à l'air.
Poids obtenu = 177 g (P.F. = 85°C P.M.= 295 - rdt = 91% en produit sec).

- Acide 6-cyclopropylméthylsulfonyl chromane 8-carboxylique.

Dans un ballon de 3 litres, on a introduit 177 g d'acide 6-cyclopropylméthylthio chromane 8-carboxylique, 780 ml d'acide acétique et 366 ml d'eau oxygénée à 110 volumes. On a chauffé ensuite au bain d'eau pendant 5 heures puis filtré la solution avec du noir et distillé une partie de l'acide acétique.

Le résidu a été repris à l'eau puis le solide obtenu a été essoré, lavé à l'eau et séché à 40°-50°C. Poids obtenu = 146 g (rdt = 82%).

-N- (1-cyclopropylméthyl 2-pyrrolidinylméthyl) 6-cyclopropylméthylsulfonyl chromane 8-carboxamide.

Dans un ballon de 1 litre, on a introduit 63 g d'acide 6-cyclopropylméthylsulfonyl chromane 8-carboxylique, 250 ml de chloroforme et 21,5 g de triéthylamine. On a refroidi à 5°C puis ajouté goutte à goutte, 23 g de chloroformiate d'éthyle, en maintenant la température entre 0° et 5°C. On a agité ensuite pendant 30 minutes à 5°C puis versé goutte à goutte, entre 5° et 10°C, 35,5 g de 1-cyclopropylméthyl 2-aminométhylpyrrolidine. On a ensuite agité 1 heure à 10°C et 2 heures en laissant remonter la température. On a distillé la majeure partie du chloroforme sous vide léger puis repris le résidu à l'eau et l'acide chlorhydrique. On a éliminé le chloroforme restant par entrainement à l'eau, dilué de manière à obtenir 600 ml de solution puis alcalinisé par de l'ammoniaque à 20% jusqu'à virage de la phénolphtaléine. L'huile formée s'est cristallisée après addition d'éther.
Le produit obtenu a été essoré, lavé à l'eau et séché à l'air. Poids obtenu : 89 g (rdt = 97%)
Les 89 g de base ont été dissous dans 210 ml d'éthanol absolu et 24 g d'acide phosphorique à 85%. Les cristaux formés ont été essorés, lavés et séchés.
On a obtenu 87 g de phosphate qui ont été dissous dans 800 ml d'eau contenant un peu de métabisulfite de sodium. La solution obtenue a été filtrée avec du noir puis alcalinisée par de l'ammoniaque à 20% en présence d'un peu d'éther.
Les cristaux formés ont été essorés, lavés à l'eau et séchés à 30°C. Les 69 g de base obtenus ont été recristallisés dans 140 ml d'isopropanol. Après essorage et séchage, on a obtenu 55 g de produit qui ont été redissous à chaud dans 110 ml d'isopropanol. La solution bouillante a été filtrée avec du noir. La base cristallisée en refroidissant, a été essorée, lavée à l'isopropanol et séchée à 30°C.
Poids obtenu : 43,5 g (P.F. = 95° - 95,5°C - rdt des purifications : 50% rdt total = 48%).

Exemple 5 : Chlorhydrate de N- (1-cyclopropylméthyl 2-pyrrolidinylméthyl) 6-éthylsulfonyl chromane 8-carboxamide.

- Acide 6-éthylsulfonyl chromane 8-carboxylique.

Dans un ballon de 3 litres, on a introduit 461 ml d'eau, 100 g de sulfite de sodium anhydre et 134 g de bicarbonate de sodium. On a chauffé jusqu'à 70°C, tout en agitant puis on a introduit peu à peu, entre 70 et 80°C, 147 g d'acide 6-chlorosulfonyl chromane 8-carboxylique. On a ensuite maintenu la température entre 70°C et 80°C pendant 2 heures.

On a refroidi la solution à 20°C puis ajouté 106 ml de lessive de soude à 30%, 530 ml d'éthanol et 249 g d'iodure d'éthyle. Le mélange a été chauffé à la température de reflux qui, de 60°C au début, s'est élevée progressivement au cours de la réaction jusqu'à 82°C après 18 à 20 heures de chauffage. Après refroidissement on a repris le mélange réactionnel à l'eau, filtré la solution avec du noir et acidifié par de l'acide chlorhydrique concentré jusqu'à virage du rouge congo. Le précipité formé a été essoré , lavé à l'eau et séché à 40°C.
Poids obtenu = 121 g (P.F. = 156° - 157°C - rdt = 84,5%).

- N (1-cyclopropylméthyl 2- pyrrolidinylméthyl) 6- éthylsulfonyl chromane 8- carboxamide.

Dans un ballon de 1 litre, on a introduit 80,5 g d'acide 6- éthylsulfonyl chromane 8- carboxylique, 440 ml d'acétone et 30 g de triéthylamine. On a refroidi à 0°C puis introduit goutte à goutte, 32,5 g de chloroformiate d'éthyle, en maintenant la température entre 0° et 5°C.
Le mélange a été agité 30 minutes à 5°C puis on a ajouté goutte à goutte en refroidissant, 46 g de 1- cyclopropylméthyl 2- aminométhyl pyrrolidine. On a agité pendant une heure en laissant remonter la température puis essoré le chlorhydrate de triéthylamine. L'acétone a été distillé en terminant sous vide jusqu'à poids constant puis le résidu a été dissous dans l'eau et dans l'acide chlorhydrique. La solution obtenue a été filtrée avec du noir puis alcalinisée par de l'ammoniaque à 20% jusqu'à virage de la phénolphtaléine.
L'huile formée a été décantée et extraite au chlorure de méthylène puis la solution chlorométhylénique a été séchée sur carbonate de potassium. Le chlorure de méthylène a été distillé en terminant sous vide jusqu'à poids constant.
Poids obtenu : 115 g (rdt = 95%).

- Chlorhydrate de N- (1- cyclopropylméthyl 2- pyrrolidinylméthyl) 6-éthylsulfonyl chromane 8- carboxamide.

On a dissous 130 g de N- (1- cyclopropylméthyl 2- pyrrolidinglméthyl) 6- éthylsulfonyl chromane 8-carboxamide dans 230 ml d'éthanol puis ajouté une solution de 12 g de gaz chlorhydrique dans 20 ml d'éthanol absolu, jusqu'à virage du rouge de méthyle.
Les cristaux formés après amorçage ont été essorés, lavés à l'éthanol absolu et séchés à l'air puis à 60°C.
Les 106 g de chlorhydrate obtenus ont été redissous à chaud dans 212 ml d'éthanol absolu. La solution bouillante a été filtrée avec du noir. Après refroidissement, les cristaux formés ont été essorés, lavés à l'éthanol absolu et à l'éther et séchés à l'air puis à 60°C.
Poids obtenu : 96 g (P.F = 182-183°C - rdt = 68%).

Exemple 6 : Chlorhydrate de N-(1-cyclopropylméthyl 2-pyrrolidylméthyl 5-éthylsulfonyl 2-méthyl 2,3-dihydrobenzofuranne 7-carboxamide

- Acide 5-éthylsulfonyl 2-méthyl 2,3-dihydrobenzofuranne 7-carboxylique.

Dans un ballon de 4 litres, on a introduit 147 g de sulfite de sodium, 196 g de bicarbonate de sodium et 870 ml d'eau. On a chauffé jusqu'à 80°C puis ajouté peu à peu, entre 70° et 80°C, 215 g d' acide 5-chlorosulfonyl 2-méthyl 2,3-dihydrobenzofuranne 7-carboxylique. On a chauffé ensuite 2 heures à 80°C jusqu'à la fin du dégagement gazeux.
On a refroidi à 20°C puis ajouté 800 ml d'éthanol, 240 ml de lessive de soude à 30% et 364 g d'iodure d'éthyle et chauffé au reflux en compensant les pertes d'iodure d'éthyle.
En 54h30, on a rajouté 86 g d'iodure d'éthyle et la température s'est élevée de 60° à 82°C.
Aprés distillation d'une partie de l'alcool, on a repris le résidu par 1,6 litre d'eau. La solution obtenu a été filtrée avec du noir puis acidifiée par de l'acide chlorhydrique concentré jusqu'à virage du rouge congo.
Le précipité formé a été essoré, lavé à l'eau, séché à 50°C, puis redissous dans 900 ml d'eau et du bicarbonate de sodium. La solution a été filtrée avec du noir puis acidifiée par de l'acide chlorhydrique concentré jusqu'à virage du rouge congo.
Le précipité a été essoré, lavé à l'eau et séché à 50°C.
Poids obtenu : 172 g (P.F. = 191°C - rdt = 82%).

- N-(1-cyclopropylméthyl 2-pyrrolidinylméthyl) 5-éthylsulfonyl 2-méthyl 2,3-dihydrobenzofuranne 7-carboxamide.

Dans un ballon de 1 litre, on a introduit 81 g d'acide 5-éthylsulfonyl 2-méthyl 2,3-dihydrobenzofuranne 7-carboxylique, 300 ml de chloroforme et 30 g de triéthylamine. On a refroidi à 0°C puis ajouté, goutte à goutte, 32 g de chloroformiate d'éthyle, en maintenant la température entre 0° et 5°C. On a agité pendant 2 heures, puis ajouté goutte à goutte, 46,5 g de 1-cyclopropylméthyl 2-aminométhylpyrrolidine, en maintenant la température entre 5° et 10°C.
On a ensuite laissé remonter la température, puis la solution a été reprise par 1600 ml d'eau et acidifiée par de l'acide acétique jusqu'à ph 4. Le chloroforme a été distillé puis la solution aqueuse restante a été filtrée avec du noir et alcalinisée par de l'ammoniaque à 20% jusqu'à virage de la phénolphtaléine.
L'huile formée a été décantée et extraite à l'éther. La solution obtenue a été séchée sur carbonate de potassium puis l'éther a été distillé en terminant sous vide jusqu'à poids constant.
Poids obtenu = 104 g (rdt = 85%).

- Chlorhydrate de N-(1-cyclopropylméthyl 2-pyrrolidinylméthyl) 5-éthylsulfonyl 2-méthyl 2,3-dihydrobenzofuranne 7-carboxamide.

On a dissous 104 g de base dans 370 ml d'acétate d'éthyle puis ajouté une solution de 9,5 g de gaz chlorhydrique dans 70 ml d'acétate d'éthyle.
Le précité de chlorhydrate a été essoré, lavé avec 50 ml d'acétate d'éthyle puis avec de l'éther et séché à l'air puis à 40°C.
Les 104 g de chlorhydrate obtenus ont été dissous dans 520 ml d'eau, puis la solution a été filtrée avec du noir et alcalinisée par de l'ammoniaque à 20% jusqu'à virage de la phénolphtaléine.
L'huile formée a été décantée et extraits à l'éther. La solution obtenue a été séchée sur carbonate de potassium puis l'éther a été distillé en terminant sous vide jusqu'à poids constant.
Les 91 g de base obtenus ont été dissous dans 320 ml d'acétate d'éthyle puis on a ajouté une solution de 8 g

de gaz chlorhydrique dans 50 ml d'acétate d'éthyle.

Le précipité formé a été essoré, lavé avec de l'acétate d'éthyle puis de l'éther et séché à l'air puis à 40°C. Poids obtenu : 96 g de produit hydraté (contenant une demi-mole d'eau). (P.F. = 137-138°C - rdt = 83%).

Exemple 7 : N- (1-cyclohexénylméthyl 2-pyrrolidinylméthyl) 6-éthylsulfonyl chromane 8-carboxamide.

- N- (2,5-dichloropentyl) 6-éthylsulfonyl chromane 8-carboxamide.

Dans un ballon de 1 litre, on a introduit 75 g d'acide 6-éthylsulfonyl chromane 8-carboxylique, 280 ml de chloroforme et 28 g de triéthylamine. On a refroidi à 0°C, puis ajouté goutte à goutte , 30 g de chloroformiate d'éthyle, en maintenant la température entre 0° et 5°C. Le mélange : ensuite été agité pendant 30 minutes entre 0° et 5°C.

Dans un ballon de 2 litres, on a introduit 54 g de chlorhydrate de 2,5-dichloropentylamine, 280 ml de chloroforme et 28 g de triéthylamine puis on a versé goutte à goutte, la solution préparée précédemment. La température s'est élevée à 27°C. La solution a ensuite été reprise à l'eau. La couche chloroformique a été décantée, séchée sur sulfate de sodium puis le chloroforme a été distillé en terminant sous vide jusqu'à poids constant. Poids obtenu : 107 g (rdt = 94%).

- N- (1-cyclohexénylméthyl 2-pyrrolidinylméthyl) 6-éthylsulfonyl chromane 8-carboxamide.

Dans un ballon de 1 litre, on a introduit 107 g de N-(2,5-dichloropentyl) 6-éthylsulfonyl chromane 8-carboxamide finement pulvérisés et 203,5 g de 1-cyclohexénylméthylamine. On a chauffé à 60°C pendant 2 heures puis laissé la solution dans une étuve à 60°C pendant 48 heures.

Après addition d'eau et de 30 ml de lessive de soude à 30%, on a distillé l'amine en excès. On a refroidi puis extrait à l'éther le précipité. Les cristaux formés ont été essorés, lavés à l'éther et séchés à l'air.
On a obtenu 47 g de produit.

D'autre part, les jus éthérés ont été évaporés et le résidu a été repris au chlorure de méthylène. La solution a été séchée sur carbonate de potassium puis le chlorure de méthylène a été distillé en terminant sous vide -jusqu'à poids constant.

Le résidu a été repris à l'éther puis les cristaux formés ont été essorés, lavés et séchés à l'air.
On a obtenu 21 g de produit supplémentaires.

Les 68 g de produit ont été redissous à chaud dans 136 ml d'isopropanol. La solution bouillante a été filtrée avec du noir puis refroidie. Les cristaux formés ont été essorés, lavés à l'isopropanol puis a l'éther et séchés à l'air puis à 35°C.

Poids obtenu : 57 g (P.F. = 91°C - rdt = 49%).

Exemple 8 : Fumarate de N-(1-cyclohexénylméthyl 2-pyrrolidinylméthyl) 6-chloro chromane 8-carboxamide

- Chlorure de 6-chloro chromane 8-carbonyle.

Dans un ballon de 1 litre, on a introduit 246 g de chlorure de thionyle et 55 g d'acide 6-chloro chromane 8-carboxylique puis on a chauffé au bain d'eau à 40° - 50°C, jusqu'à dissolution. On a refroidi un peu, rajouté 55 g d'acide 6-chloro chromane 8-carboxylique puis chauffé de nouveau jusqu'à dissolution.
On a ensuite chauffé au bain d'eau pendant 1 heure puis distillé l'excès de chlorure de thionyle sous vide.

On a obtenu 109 g de chlorure d'acide (rdt = 91%).

- N-(1-cyclohexénylméthyl 2-pyrrolidinylméthyl) 6-chloro chromane 8-carboxamide.

Dans un ballon de 2 litres, on a introduit 101 g de 1-cyclohexénylméthyl 2-aminométhyl pyrrolidine et 200 ml de méthyléthylcétone. On a refroidi à 5°C, puis ajouté goutte à goutte une solution de 109 g de chlorure de 6-chloro chromane 8-carbonyle dans 500 ml de méthyléthylcétone, en maintenant la température entre 0° et 5°C.
Le mélange a ensuite été laissé 1 heure à 5°C et une nuit à la température ambiante.
Le produit obtenu'a été essoré, lavé à la méthyléthylcétone et séché à 40°C.
Les 195 g de produit obtenus ont été redissous à chaud dans 2 litres d'eau. La solution a été filtrée avec du noir puis alcalinisée par de l'ammoniaque à 20% jusqu'à virage de la phénolphtaléine. L'huile formée a été extraite à l'éther, puis la phase éthérée a été séchée sur carbonate de potassium et l'éther distillé en terminant sous vide jusqu'à poids constant.

Poids obtenu : 177 g (rdt = 97%).

- Fumarate de N-(1-cyclohexénylméthyl 2-pyrrolidinylméthyl) 6-chloro chromane 8-carboxamide.

On a dissous à chaud 197 g de N-(1-cyclohexénylméthyl 2-pyrrolidinylméthyl) 6-chloro chromane 8-carboxamide dans 450 ml d'éthanol absolu et 59 g d'acide fumarique. Les cristaux formés après refroidissement ont été essorés, lavés à l'éthanol et séchés à 40°C.
On a obtenu 218 g de fumarate qui ont été recristallisés dans 650 ml d'éthanol à 95%.
Les cristaux ont été essorés, lavés et séchés à 40°C.
Poids obtenu = 196 g (P.F. = 166° - 167°C - rdt = 77%).

Exemple 9 : N-(1-cyclohexénylméthyl 2-pyrrolidinylméthyl) 5-chloro 2,3-dihydrobenzofuranne 7-carboxamide.

Acide 5-chloro 2,3-dihydrobenzofuranne 7-carboxilique.
Dans un ballon de 1 litre, on a introduit 34 g d'acide 2,3-dihydrobenzofuranne 7-carboxylique et 204 ml d'acide acétique puis on a fait passer un courant de chlore dans la suspension obtenue, tout en refroidissant dans un bain glace-sel. Au bout de 2 à 3 heures, la suspension s'est fluidifiée. 40 g de chlore ont été absorbés.
On a alors éliminé l'excès de chlore par aspiration sous vide, en intercalant un piège contenant de la lessive de soude. La majeure partie de l'acide acétique a été éliminée sous vide puis le résidu repris à l'eau glacée.
L'acide obtenu a été essoré, lavé à l'eau et séché à 50°C en étuve ventilee.
On a obtenu 28,5 g de produit qui ont été repris dans 30 ml d'éther. On a essoré, lavé et séché à l'air puis à 40°C.
Poids obtenu : 20 g (P.F. =226°C rdt = 48%).

- N-(1-cyclohexénylméthyl 2-pyrrolidinylméthyl) 5-chloro 2,3-dihydrobenzofuranne 7-carboxamide.

Dans un ballon de 1 litre, on a introduit 32 g d'acide 5-chloro 2,3-dihydrobenzofuranne 7-carboxylique, 130 ml de chloroforme et 16 g de triéthylamine. On a refroidi à 0°- 5°C puis ajouté goutte à goutte, 17,5 g de chloroformiate d'éthyle et agité le mélange pendant 1 heure 30 On a versé ensuite, goutte à goutte, entre 5 et 10°C, une solution de 34 g de 1-cyclohexénylméthyl 2-aminométhylpyrrolidine dans 68 ml de chloroforme, puis agité le mélange pendant 1 heure à 5°C et 1 heure à la température ambiante.
On a distillé le chloroforme sous vide léger puis repris le résidu à l'eau et l'acide acétique nécessaire pour ajuster le pH à 4. La solution obtenue a été filtrée avec du noir et alcalinisée par de la lessive de soude à 30% jusqu'à virage de la phénolphtaléine. Le précipité formé a été essoré, lavé à l'eau et séché dans une étuve ventilée à 40°C. On a obtenu 60 g de base qui ont été recristallisés dans 150 ml d'éthanol à 90%.
Poids obtenu : 39 g (P.F. = 104°C rdt = 65%).

Exemple 10 : Ethane disulfonate neutre de N-(1-cyclohexénylméthyl 2-pyrrolidinylméthyl) 5-chloro 2-méthyl 2,3-dihydrobenzofuranne 7-carboxamide.

- N-(1-cyclohexénylméthyl 2-pyrrolidinylméthyl) 5-chloro 2-méthyl 2,3-dihydrobenzofuranne 7-carboxamide.

Dans un ballon de 1 litre, on a introduit 51 g d'acide 5-chloro 2-méthyl 2,3-dihydrobenzofuranne 7-carboxylique, 240 ml de chloroforme et 24,5 g de triéthylamine. On a refroidi à 0°C puis ajouté goutte à goutte, 26 g de chloroformiate d'éthyle, en maintenant la température entre 0° et 5°C.
On a agité le mélange pendant 2 heures, puis versé goutte à goutte, entre 5° et 10°C, 46,5 g de 1-cyclohexénylméthyl 2-aminométhyl pyrrolidine. On a agité pendant 30 minutes en laissant remonter la température. On a alors repris la solution à l'eau et ajusté le pH à 4 par addition d'acide acétique. Le chloroforme a été distillé sous vide puis la solution obtenue a été filtrée avec du noir et alcalinisée par de l'ammoniaque à 20% jusqu'à virage de la phénolphtaléine.
L'huile formée a été extraite à l'éther puis la solution éthérée a été séchée sur sulfate de sodium et l'éther a été distillé en terminant sous vide jusqu'à poids constant.
Les 83 g de base obtenus ont été dissous dans 332 ml d'éthanol absolu. On a ajouté 24,8 g d'acide fumarique puis chauffé jusqu'à dissolution. Les cristaux formés après refroidissement ont été essorés, lavés à l'éthanol absolu et séchés à 50°C.
Les 85 g de fumarate obtenus ont été recristallisés dans 500 ml d'eau. Le précipité formé après refroidissement a été essoré, lavé à l'eau et séché, puis redissous dans 2 litres d'eau.

La solution obtenue a été alcalinisée par de l'ammoniaque à 20% jusqu'à virage de la phénolphtaléine.
L'huile formée a été extraite à l'éther puis la solution éthérée a été séchée sur sulfate de sodium et l'éther a été distillé en terminant sous vide jusqu'à poids constant.
On a obtenu 56 g d'un produit huileux (rdt = 60%).

- Ethane disulfonate neutre de N-(1-cyclohexénylméthyl 2-pyrrolidinylméthyl) 5-chloro 2-méthyl 2,3-dihydrobenzofuranne 7-carboxamide.

On a dissous 56 g de base dans 250 ml d'éthanol absolu puis ajouté 33 g d'acide éthane disulfonique contenant 18% d'eau. On a tiédi le mélange jusqu'à dissolution. Après refroidissement, les cristaux formés ont été essorés, lavés à l'éthanol et séchés à 50°C.
Les 57 g de produit obtenus ont été redissous à froid dans 570 ml d'eau puis la solution a été concentrée au bain marie.

Les cristaux formés a-près refroidissement ont été séchés à l'étuve. Après pulvérisation, le produit a été laiseé à l'air jusqu'à ce que son poids soit devenu stable.
Le produit contient 2 moles d'eau.
Poids obtenu : 53 g de produit hydraté (P.F. = 85° - 90°C rdt = 71%).

Les composés de l'invention ont fait l'objet d'une étude toxicologique et pharmacologique.

Leur toxicité aiguë a été étudiée chez la souris, les composés étant administrés par voie intraveineuse, sous-cutanée, intrapéritonéale et orale.

Les doses entraînant la mortalité de 50% des animaux ($DI_{50}$) ont été déterminées par la méthode de Bliss, fournissant les résultats suivants :

| Doses léthales 50 exprimées en mg/kg | | | | |
|---|---|---|---|---|
| Composé | voie I.V. | voie S.C. | voie I.P. | voie orale |
| ex 1 | 30,5 - 35,2 | 316 - 318 | 134 - 152 | 259 - 295 |
| ex 2 | 42,6 - 43,7 | 406 - 408 | 167 - 172 | 342 - 354 |
| ex 3 | 14 - 16 | 250 | 117 - 125 | 166 - 170 |
| ex 4 | 37,9 - 46,1 | 291 - 312 | 163 - 165 | 408 - 412 |
| ex 5 | 60,9 - 63,3 | 415 - 435 | 239 - 266 | 479 - 532 |
| ex 6 | 68,8 - 70,2 | 440 - 462 | 211 - 213 | 390 - 391 |
| ex 7 | 28 - 28,6 | 141 - 145 | 125 - 127 | 149 - 163 |
| ex 8 | 18,6 - 18 | 0% à 900mg/kg | 120 - 120 | 422 - 473 |
| ex 9 | 24,8 | 412 - 434 | 69,6 - 83,3 | 350 - 354 |
| ex 10 | 31,1 - 34,7 | 1407 | 232 - 234 | 561 - 521 |

Selon l'état de la connaissance pharmacologique, la structure chimque des composés de l'invention suggérait qu'ils puissent avoir une propriété neuroleptique.

En conséquence, une étude de leur action sur le système nerveux central a été conduite en appliquant des tests classiques destinés à mettre en évidence cette propriété. Ainsi ont été notamment recherchés un effet inhibiteur de la motricité spontanée chez la souris, un pouvoir cataleptigène chez le rat, des antagonismes vis à vis de certains effets comportementaux provoqués par l'apomorphine et l'amphétamine. Sur la motricité spontanée chez la souris, un'effet inhibiteur des composés a été effectivement observé et enregistré par un procédé photoélectique selon une technique proche de celle de Winter et Flataker (J. Pharmacol. Exp. Ther. 1951, 101, 156 - 162) et par actívographie utilisant un appareil Animex.

Les composés ont été administrés par voie intrapéritonéale ou orale, respectivement 15 ou 60 minutes avant les enregistrements selon la méthode retenue.

Les valeurs des doses inhibitrices 50 % de la motricité ($DI_{50}$) des composés sont rassemblés dans le tableau suivant :

| Inhibition de la motricité spontanée chez la souris | | | | |
|---|---|---|---|---|
| | Test de Winter et Flataker | | Test de l'activographe Animex | |
| Composé | $DI_{50}$ I.P. (mg/kg) | $DI_{50}$ P.O. (mg/kg) | $DI_{50}$ I.P. (mg/kg) | $DI_{50}$ P.O. (mg/kg) |
| ex 1 | 1,8 | 12,4 - 13,2 | 2,15 - 3,4 | 19,2 - 22,4 |
| ex 2 | 6,2 | 31,5 | 7,5 | 31,5 |
| ex 3 | 1,21 | 17,3 | 0,55 | 8,5 |
| ex 4 | 15,4 | 55 | 11,4 | 40 |
| ex 5 | 9,2 | 33,9 | 12,8 | 35,8 |
| ex 6 | 3,2 | 31,5 | 3 | 23,4 |
| ex 7 | 1,3 | 16,6 | 1,22 | 16,6 |
| ex 8 | 1,96 | 20,9 | 3 | 22,7 |
| ex 9 | 2,1 | 27 | 1,7 | 25 |
| ex 10 | 3,5 | 43,4 | 2,2 | 23,2 |

La fonction cataleptigène des composés de l'invention a été étudiée chez le rat. Chacun des composés a été administré par voie sous cutanée, à doses croissantes, à des groupes de 10 rats (un groupe pour chaque dose). Chaque groupe a été observé pendant 7 heures et le pourcentage des animaux présentant une catalepsie a été établi toutes les heures, le critère de l'état cataleptique étant l'immobilité pendant 30 secondes, les membres antérieurs du rat étant écartés et placés sur des cubes de bois de 4 cm de hauteur. La dose entraînant la catalepsie chez 50% des animaux ($DE_{50}$) a été déterminée graphiquement, au maximum de l'effet.

Les valeurs obtenues sont indiquées dans le tableau suivant :

| Catalepsie chez le rat par voie sous-cutanée - en mg/kg | | | |
|---|---|---|---|
| Composé | $DE_{50}$ | Composé | $DE_{50}$ |
| ex 1 | 6,6 | ex 7 | 1,66 |
| ex 2 | 17,6 | ex 8 | 1,5 |
| ex 3 | 2,3 | ex 9 | 2,7 |
| ex 6 | 20,7 | ex 10 | 1,35 |

L'apomorphine et l'amphétamine provoquent chez le rat des mouvements stéréotypés qui sont antagonisés par les neuroleptiques. Différentes doses d'apomorphine administrée par différentes voies ont été appliquées pour produire ces mouvements :
1,25 mg/kg par voie intraveineuse comme dans le test préconisé par Janssen (Arzn. Forsch.1960, 10, 1003 - 1005), le composé à étudier étant administré, par voie sous-cutanée, 60 minutes avant et l'observation de l'antagonisme étant faite 20 minutes après l'administration de l'apomorphine ou 0,50 mg/kg par voie sous-cutanée, selon une technique dérivée de celle de Puech (Eur. J. Pharmacol. 1978, 50, 291 - 300), le composé étant administré par voie intrapéritonéale, 30 minutes avant et l'effet étant observé 20 minutes après l'administration de l'apomorphine.
Dans le test utilisant la dexamphétamine, réalisé selon la technique de Janssen (Arz. Forsch. 1961, 11, 932 - 938), 10 mg/kg de dexamphétamine ont été injectés par voie intraveineuse, le produit à étudier étant injecté simultanément par voie sous-cutanée et l'effet étant mesuré 60 minutes après ces injections.
Ces différentes conditions expérimentales ont permis d'établir les doses qui antagonisent de 50 % les stéréotypies ($DI_{50}$), jugées d'après les diverses composantes des mouvements observées.
Les valeurs de ces $DI_{50}$ sont rassemblées dans le tableau suivant :

| Antagonisme des effets de l'apomorphine et de l'amphétamine chez le rat. | | | |
|---|---|---|---|
| Composé | Stéréotypies à l'apomorphine (1,25 mg/kg IV) $DI_{50}$ S.C. (mg/kg) | stéréotypies à l'apomorphine (0,5 mg/kg SC) $DI_{50}$ I.P. (mg/kg) | Test de Jansen à l'amphétamine $DI_{50}$ S.C. (mg/kg) |
| ex 1 | 0,375 | 0,64 - 0,65 | 0,285 |
| ex 2 | 0,73 | 2,5 - 2,7 | 0,77 |
| ex 3 | 0,084 | | 0,078 |
| ex 4 | 13,2 | 9,4 - 11,1 | 4,2 |
| ex 5 | 9,5 | 7,1 - 8,1 | 3,9 |
| ex 6 | 3,6 | 1,2 - 1,6 | 1,1 |
| ex 7 | 0,134 | 0,18 - 0,23 | 0,066 |
| ex 8 | 0,22 | 0,38 - 0,44 | 0,15 |
| ex 9 | 0,29 | 0,31 - 0,41 | 0,12 |
| ex 10 | 0,41 | 0,58 - 0,59 | 0,18 |

Un autre test utilisant l'apomorphine a été appliqué, basé sur l'observation du comportement de verticalisation qu'elle provoque chez la souris, comportement qui est antagonisé par les neuroleptiques, selon Puech (Eur. J. Pharmacol. 1978, 50, 291 - 300).
Le composé a été administré par voie intrapéritonéale 30 minutes avant l'apomorphine (1 mg/kg par voie souscutanée) et l'antagonisme a été apprécié 45 à 50 minutes après l'administration du composé. Les doses inhibitrices 50% de ce comportement ($DI_{50}$) déterminées dans ces conditions, sont indiquées dans le tableau suivant :

| Antagonisme du comportement de verticalisation induit par l'apomorphine chez la souris. | | | |
|---|---|---|---|
| composé | $DI_{50}$ I.P. (mg/kg) | Composé | $DI_{50}$ I.P. (mg/kg) |
| ex. 1 | 0,55 - 0,63 | ex 7 | 0,105 - 0,126 |
| ex. 2 | 1,20 - 1,24 | ex 8 | 0,32 - 0,35 |
| ex. 4 | 3,2 - 3,5 | ex 9 | 0,27 - 0,29 |
| ex. 5 | 3,3 - 4,1 | ex 10 | 0,28 - 0,34 |
| ex. 6 | 0,40 - 0,55 | | |

Les résultats obtenus par les tests décrits ci-dessus démontrent que les composés de l'invention sont capables, à des doses parfois très faibles, d'inhiber la motricité spontanée chez la souris, de provoquer la catalepsie chez le rat et d'antagoniser certains comportements (stéréotypies, verticalisation) provoqués par l'apomorphine ou l'amphétamine chez la souris ou le rat. En conséquence, les composés de l'invention possèdent des caractéristiques pharmacologiques typiques des neuroleptiques, certains pouvant être très puissants à cet égard. Les tests cliniques réalisés avec les composés de l'invention ont confirmés leur potentiel neuroleptique révélé par la pharmacologie.

**Revendications**

**Revendications pour les Etats contractants suivants : BE, CH, DE, FR, GB, IT, LI, LU, NL, SE**

1. Dérivés de dihydrobenzofuranne - et de chromane - carboxamide, de formule (I) :

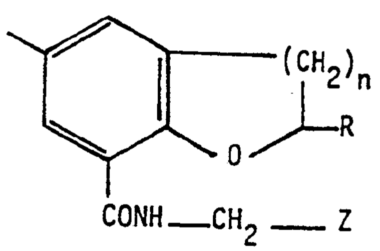

(I)

dans laquelle

- R représente un atome d'hydrogène ou un groupe méthyle
- n est égal à 1 ou 2
- Z est un groupe de formule

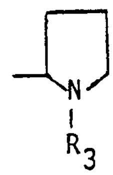

dans laquelle $R_3$ représente un groupe cycloalkylalkyle ou cycloalcénylalkyle,

– Y est un atome de chlore ou un groupe cycloalkylméthylsulfonyle, ainsi que leurs sels d'addition d'acides pharmacologiquement acceptables et leurs isomères optiques.

2. N-(1-cyclohexénylméthyl 2-pyrrolidinylméthyl) 5-méthylsulfamoyl 2-méthyl 2,3-dihydrobenzofuranne 7-carboxamide.

3. N-(1-cyclohexénylméthyl 2-pyrrolidinylméthyl) 6-méthylsulfamoyl chromane 8-carboxamide.

4. Selon la revendication 1, le N-(1-cyclohexénylméthyl 2-pyrrolidinylméthyl) 5-cyclopropylméthylsulfonyl 2-méthyl 2,3-dihydrobenzofuranne 7-carboxamide.

5. Selon la revendication 1, le N-(1-cyclopropylméthyl 2-pyrrolidinylméthyl) 6-cyclopropylméthylsulfonyl chromane 8-carboxamide.

6. N-(1-cyclopropylméthyl 2-pyrrolidinylméthyl) 6-éthylsulfonyl chromane 8-carboxamide.

7. N-(1-cyclopropylméthyl 2-pyrrolidinylméthyl) 5-éthylsulfonyl 2-méthyl 2,3-dihydrobenzofuranne 7-carboxamide.

8. N-(1-cyclohexénylméthyl 2-pyrrolidinylméthyl) 6-éthylsulfonyl chromane 8-carboxamide.

9. Selon la revendication 1, le N-(1-cyclohexénylméthyl 2-pyrrolidinylméthyl) 6-chloro chromane 8-carboxamide.

10. Selon la revendication 1, le N-(1-cyclohexénylméthyl 2-pyrrolidinylméthyl) 5-chloro 2,3-dihydrobenzofuranne 7-carboxamide.

11. Selon la revendication 1, le N-(1-cyclohexénylméthyl 2-pyrrolidinylméthyl) 5-chloro 2-méthyl 2,3-dihydrobenzofuranne 7-carboxamide.

12. Procédé de préparation des composés de formule (I) selon la revendication 1, qui consiste : à traiter un acide de formule (II):

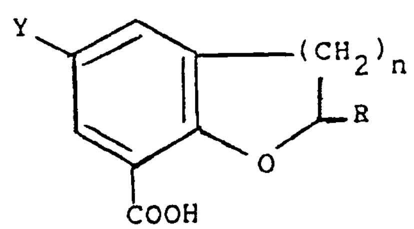

(II)

dans laquelle R, Y et n sont définis comme dans la revendication 1, ou l'un de ses dérivés réactifs, par une amine de formule (III):

$$H_2N-CH_2-Z \qquad (III)$$

dans laquelle Z est défini comme dans la revendication 1, puis bien à traiter un acide de formule (II) ou l'un de ses dérivés réactifs, par une dihaloalkylamine de formule (IV) :

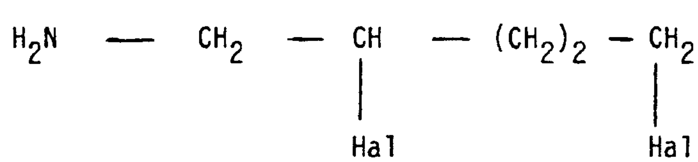

(IV)

dans laquelle Hal représente un atome d'halogène, puis à traiter le composé obtenu, de formule (V):

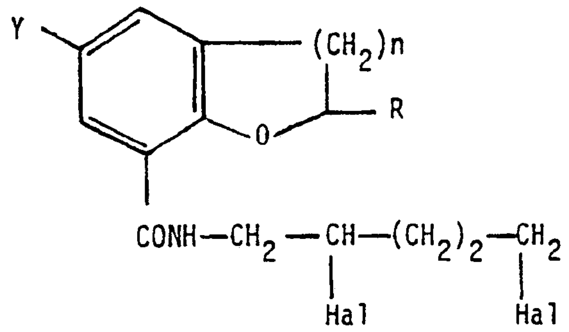

(V)

dans laquelle R, Y, n et Hal sont définis comme précédemment, par une amine de formule (VI)

$H_2N—R_3$ (VI)

dans laquelle $R_3$ est défini comme dans la revendication 1.

13. Procédé de préparation du composé selon la revendication 2, qui consiste à traiter le chlorure de 5-méthylsulfamoyl 2-méthyl 2,3-dihydrobenzofuranne 7-carbonyle par la 1-cyclohexénylméthyl 2-aminométhylpyrrolidine.

14. Procédé de préparation du composé selon la revendication 3, qui consiste à traiter l'acide 6-méthylsulfamoyl chromane 8-carboxylique par la 1-cyclohexénylméthyl 2-aminométhyl pyrrolidine, en présence de chloroformiate d'éthyle.

15. Procédé de préparation des composés selon les revendications 6 ou 7, qui consiste à traiter l'acide 6-éthylsulfonyl chromane 8-carboxylique ou l'acide 5-éthylsulfonyl 2-méthyl 2,3-dihydrobenzoturanne 7-carboxylique par la 1-cyclopropylméthyl 2-aminométhyl pyrrolidine, en présence de chloroformiate d'éthyle.

16. procédé de préparation du composé selon la revendication 8, qui consiste à traiter l'acide 6-éthylsulfonyl chromane 8-carboxylique par la 2,5-dichloropentylamine, en présence de chloroformiate d'éthyle, puis à traiter le N-(2,5-dichloropentyl) 6-éthylsulfonyl chromane 8-carboxamide obtenu, par la 1-cyclohexényl-méthylamine.

17. Composés selon l'une des revendications 1 à 11, utilisés comme médicaments, en particulier comme neuroleptiques.

18. Composition pharmaceutique comprenant un composé selon l'une des revendications 1 à 11, comme principe actif, et un excipient pharmaceutiquement acceptable.

19. En tant qu'intermédiaires de synthèse utilisés, selon le procédé de la revendication 12, pour la préparation des composés de formule (I), les composés de formule (V) :

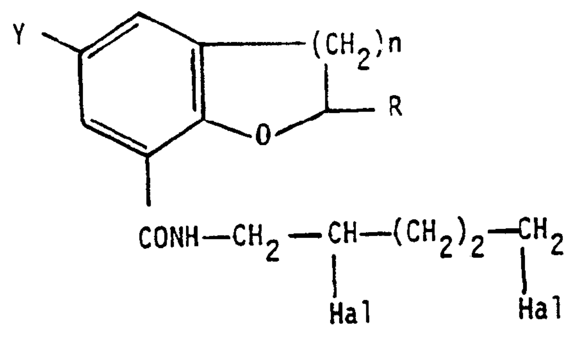

(V)

dans laquelle R, Y et n sont définis comme dans la revendication 1 et Hal est un atome d'halogène.

20. En tant qu'intermédiaire de synthèse utilisé selon le procédé de la revendication 16, le N-(2,5-dichloropentyl) 6-éthylsulfonyl chromane 8-carboxamide.

**Revendications pour les Etats contractants suivants : AT, ES, GR**

1. Procédé de préparation de dérivés de dihydrobenzofuranne- et de chromane-carboxamide de formule (I) :

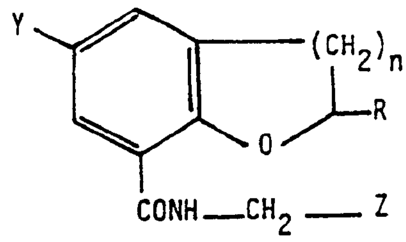

(I)

dans laquelle :

- R represente un atome d'hydrogène ou un groupe méthyle,
- n est égal à 1 ou 2
- Z est un groupe de formule:

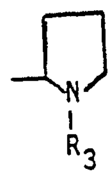

dans laquelle $R_3$ représente un groupe cycloalkylalkyle ou cycloalcénylalkyle,

- Y est un atome de chlore ou un groupe cycloalkylméthylsulfonyle, ainsi que de leurs sels d'addition d'acides pharmacologiquement acceptables et de leurs isomères optiques,

qui consiste à traiter un acide de formule (II) :

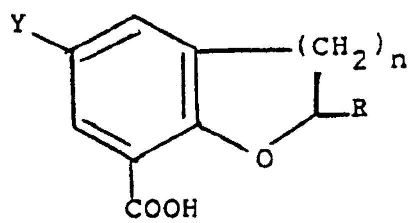

(II)

dans laquelle R, Y et n sont définis comme dans la formule (I), ou l'un de ses dérivés réactifs, par une amine de formule (III):

$$H_2N - CH_2 - Z \qquad (III)$$

dans laquelle Z est défini comme dans la formule (I),
ou bien, à traiter un acide de formule (II) ou l'un de ses dérivés réactifs, par une dihaloalkylamine de formule (IV) :

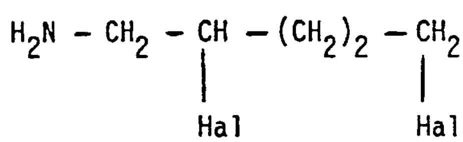

(IV)

dans laquelle Hal représente un atome d'halogène, puis à traiter le composé obtenu, de formule (V) :

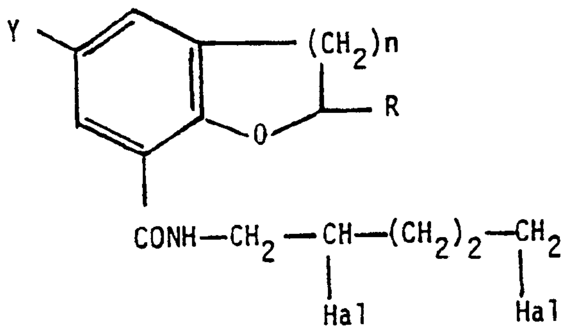

(V)

dans laquelle Hal, R, Y et n sont définis comme précédemment, par une amine de formule (VI):

$H_2N - R_3$ (VI)

dans laquelle $R_3$ est défini comme précédemment.

2. Procédé de préparation du N-(1-cyclohexénylméthyl 2-pyrrolidinylméthyl) 5-méthylsulfamoyl 2-méthyl 2,3-dihydrobenzofuranne 7-carboxamide, qui consiste à traiter le chlorure de 5-méthylsulfamoyl 2-méthyl 2,3-dihydrobenzofuranne 7-carbonyle par la 1-cyclohexénylméthyl 2-aminométhyl pyrrolidine.

3. procédé de préparation du N-(1-cyclohexénylméthyl 2-pyrrolidinylméthyl) 6-méthylsulfamoyl chromane 8-carboxamide qui consiste à traiter l'acide 6-méthylsulfamoyl chromane 8-carboxylique par la 1-cyclohexényIméthyl 2-aminométhyl pyrrolidine, en présence de chloroformiate d'éthyle.

4. procédé de préparation du N-(1-cyclopropylméthyl 2-pyrrolidinylméthyl) 6-éthylsulfonyl chromane 8-carboxamide ou du N-(1-cyclopropylméthyl 2-pyrrolidinylméthyl) 5-éthylsulfonyl 2-méthyl 2,3-dihydrobenzofuranne 7-carboxamide, qui consiste à traiter l'acide 6-éthylsulfonyl chromane 8-carboxylique ou l'acide 5-éthylsulfonyl 2-méthyl 2,3-dihydrobenzofuranne 7-carboxylique par la 1-cyclopropylméthyl 2-aminométhylpyrrolidine, en présence de chloroformiate d'éthyle.

5. Procédé de préparation du N-(1-cyclohexénylméthyl 2-pyrrolidinyl méthyl) 6-éthylsulfonyl chromane 8-carboxamide, qui consiste à traiter l'acide 6-éthylsulfonyl chromane 8-carboxylique par la 2,5-dichloropentylamine, en présence de chloroformiate d'éthyle, puis à traiter le N-(2,5-dichloropentyl) 6-éthylsulfonyl chromane 8-carboxamide obtenu, par la 1-cyclohexénylméthylamine.

## Patentansprüche

### Patentansprüche für folgende Vertragsstaaten : BE, CH, DE, FR, GB, IT, LI, LU, NL, SE

1. Derivate von Dihydrobenzofuran- bzw. Chromancarboxamid der Formel (I)

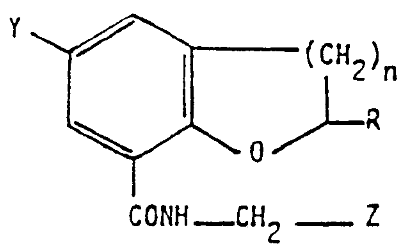

(I)

in der:

- R ein Wasserstoffatom oder eine Methylgruppe darstellt,
- n gleich 1 oder 2 ist,
- Z eine Gruppe der Formel:

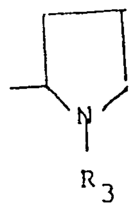

ist, in der $R_3$ eine Cycloalkylalkyl- oder Cycloalkenylalkylgruppe ist,

– Y ein Chloratom oder eine Cycloalkylmethylsulfonylgruppe ist,

sowie ihre Additionssalze mit pharmakologisch verträglichen Säuren und optischen Isomeren.

2. N-(1-Cyclohexenylmethyl-2-pyrrolidinylmethyl)-5-methylsulfamoyl-2-methyl-2,3-dihydrobenzofuran-7-carboxamid.

3. N-(1-Cyclohexenylmethyl-2-pyrrolidinylmethyl)-6-methylsulfamoyl-chroman-8-carboxamid.

4. N-(1-Cyclohexenylmethyl-2-pyrrolidinylmethyl)-5-cyclopropylmethylsulfonyl-2-methyl-2,3-dihydrobenzofuran-7-carboxamid gemäß Anspruch 1.

5. N-(1-Cyclopropylmethyl-2-pyrrolidinylmethyl)-6-cyclopropylmethylsulfonyl-chroman-8-carboxamid gemäß Anspruch 1.

6. N-(1-Cyclopropylmethyl-2-pyrrolidinylmethyl)-6-ethylsulfonyl-chroman-8-carboxamid.

7. N-(1-Cyclopropylmethyl-2-pyrrolidinylmethyl)-5-ethylsulfonyl-2-methyl-2,3-dihydrobenzofuran-7-carboxamid.

8. N-(1-Cyclohexenylmethyl-2-pyrrolidinylmethyl)-6-ethylsulfonyl-chroman-8-carboxamid.

9. N-(1-Cyclohexenylmethyl-2-pyrrolidinylmethyl)-6-chlorchroman-8-carboxamid gemäß Anspruch 1.

10. N-(1-Cyclohexenylmethyl-2-pyrrolidinylmethyl)-5-chlor-2,3-dihydrobenzofuran-7-carboxamid gemäß Anspruch 1.

11. N-(1-Cyclohexenylmethyl-2-pyrrolidinylmethyl)-5-chlor-2-methyl-2,3-dihydrobenzofuran-7-carboxamid gemäß Anspruch 1.

12. Verfahren zur Herstellung der Verbindungen der Formel (I) gemäß Anspruch 1, dadurch gekennzeichnet, daß eine Säure der Formel (II):

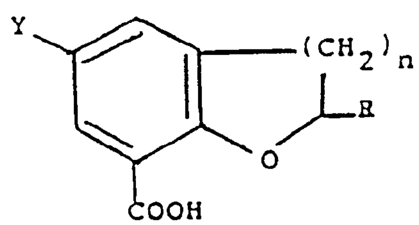

(II)

(II)

in der R, Y und n wie in Anspruch 1 definiert sind, oder ein reaktives Derivat derselben mit einem Amin der Formel (III):

$H_2N$-$CH_2$-Z (III)

in der Z wie in Anspruch 1 definiert ist, behandelt wird, oder daß eine Säure der Formel (II) oder ein reaktives Derivat derselben mit einem Dihalogenalkylamin der Formel (IV)

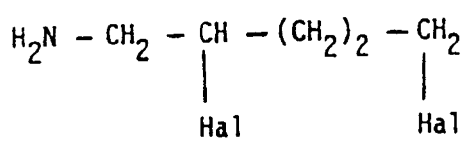

(IV)

in der Hal ein Halogenatom darstellt, behandelt wird und anschließend die erhaltene Verbindung der Formel V:

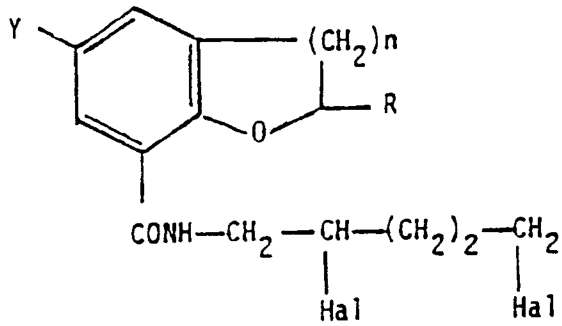

(V)

in der R, Y, n und Hal wie oben definiert sind, mit einem Amin der Formel (VI)

$H_2N\text{-}R_3$ (VI)

in der $R_3$ wie in Anspruch 1 definiert ist, behandelt wird.

13. Verfahren zur Herstellung der Verbindung gemäß Anspruch 2, dadurch gekennzeichnet, daß 5-Methylsulfamoyl-2-methyl-2,3-dihydrobenzofuran-7-carbonylchlorid mit 1-Cyclohexenylmethyl-2-aminomethylpyrrolidin behandelt wird.

14. Verfahren zur Herstellung der Verbindung gemäß Anspruch 3, dadurch gekennzeichnet, daß 6-Methylsulfamoyl-chroman-8-carbonsäure in Anwesenheit von Chlorameisensäureethylester mit 1-Cyclohexenylmethyl-2-aminomethyl-pyrrolidin behandelt wird.

15. Verfahren zur Herstellung der Verbindungen gemäß den Ansprüchen 6 oder 7, dadurch gekennzeichnet, daß 6-Ethylsulfonyl-chroman-8-carbonsäure oder 5-Ethylsulfonyl-2-methyl-2,3-dihydrobenzofuran-7-carbonsäure in Anwesenheit von Chlorameisensäureethylester mit 1-Cyclopropylmethyl-2-aminomethyl-pyrrolidin behändelt wird.

16. Verfahren zur Herstellung der Verbindung gemäß Anspruch 8, dadurch gekennzeichnet, daß 6-Ethylsulfönyl-chroman-8-carbonsäure in Anwesenheit von Chlorameisensäure mit 2,5-Dichlorpentylamin behandelt wird und anschließend das erhaltene N-(2,5-Dichlorpentyl)-6-ethylsulfonyl-chroman-8-carboxamid mit 1-Cyclohexenyl-methylamin behandelt wird.

17. Verbindungen gemäß einem der Ansprüche 1 bis 11, verwendet als Medikamente, insbesondere als Neuroleptika.

18. Pharmazeutische Zusammensetzung, umfassend eine Verbindung gemäß einem der Ansprüche 1 bis 11 als Wirkstoff und einen pharmazeutisch verträglichen Exzipienten.

19. Verbindungen der Formel (V):

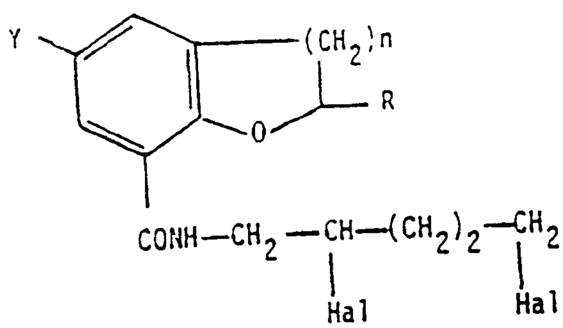

(V)

in der R, Y und n wie in Anspruch 1 definiert sind und Hal ein Halogenatom ist, als Zwischenprodukte zur Herstellung der Verbindungen der Formel (I) gemäß dem Verfahren des Anspruchs 12.

20. N-(2,5-Dichlorpentyl)-6-ethylsulfonyl-chroman-8-carboxamid, als Zwischenprodukt der Synthese gemäß dem Verfahren des Anspruchs 16.

**Patentansprüche für follgende Vertragsstaaten: AT, ES, GR**

1. Verfahren zur Herstellung von Derivaten von Dihydrobenzofuran- und Chromancarboxamid der Formel (I)

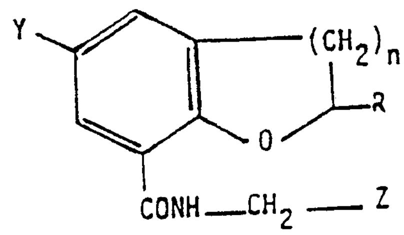

(I)

in der:
- R ein Wasserstoffatom oder eine Methylgruppe darstellt,
- n gleich 1 oder 2 ist,
- Z eine Gruppe der Formel:

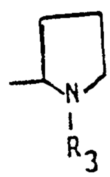

ist,
in der $R_3$ eine Cycloalkylalkyl- oder Cycloalkenylalkylgruppe darstellt,
- Y ein Chloratom oder eine Cycloalkylmethylsulfonylgruppe ist,

sowie ihrer Additionssalze mit pharmakologisch verträglichen Säuren und optischen Isomeren, dadurch gekennzeichnet, daß eine Säure der Formel (II):

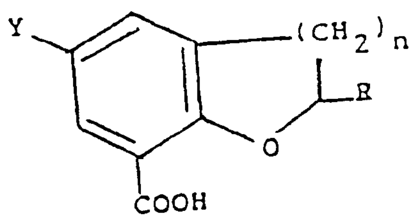

(II)

in der R, Y und n wie in der Formel (I) definiert sind, oder ein reaktives Derivat derselben mit einem Amin der Formel (III):

$$H_2N - CH_2 - Z \qquad (III)$$

in der Z wie in Formel (I) definiert ist, behandelt wird, oder daß eine Säure der Formel (II) oder ein reaktives Derivat derselben mit einem Dihalogenalkylamin der Formel (IV):

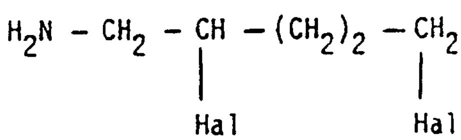

(IV)

in der Hal ein Halogenatom darstellt,
behandelt wird und anschließend die erhaltene Verbindung der Formel (V):

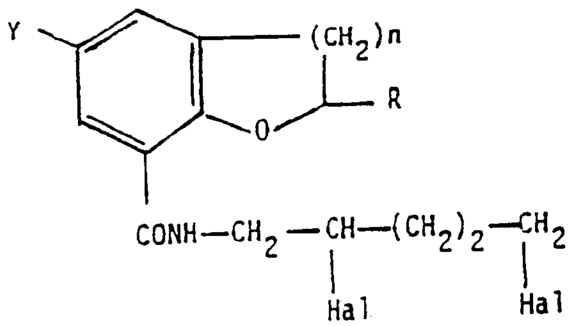

(V)

in der Hal, R, Y und n wie oben definiert sind, mit einem Amin der Formel (VI)

$H_2N\text{-}R_3$ (VI)

in der $R_3$ wie oben in Anspruch 1 definiert ist, behandelt wird.

2. Verfahren zur Herstellung von N-(1-Cyclohexenylmethyl-2-pyrrolidinylmethyl)-5-methylsulfamoyl-2-methyl-2,3-dihydrobenzofuran-7-carboxamid, dadurch gekennzeichnet, daß 5-Methylsulfamoyl-2-methyl-2,3-dihydrobenzofuran-7-carbonylchlorid mit 1-Cyclohexenylmethyl-2-aminomethyl-pyrrolidin behandelt wird.

3. Verfahren zur Herstellung von N-(1-Cyclohexenylmethyl-2-pyrrolidinylmethyl)-6-methylsulfamoyl-chroman-8-carboxamid, dadurch gekennzeichnet, daß 6-Methylsulfamoylchroman-8-carbonsäure in Anwesenheit von Chlorameisensäureethylester mit 1-Cyclohexenylmethyl-2-aminomethylpyrrolidin behandelt wird.

4. Verfahren zur Herstellung von N-(1-Cyclopropylmethyl-2-pyrrolidinylmethyl)-6-ethylsulfonyl-chroman-8-carboxamid oder von N-(1-Cyclopropylmethyl-2-pyrrolidinylmethyl)-5-ethylsulfonyl-2-methyl-2,3-dihydrobenzofuran-7-carboxamid, dadurch gekennzeichnet, daß 6-Ethylsulfonylchroman-8-carbonsäure oder 5-Ethylsulfonyl-2-methyl-2,3-dihydrobenzofuran-7-carbonsäure in Anwesenheit von Chlorameisensäureethylester mit 1-Cyclopropylmethyl-2-aminomethylpyrrolidin behandelt wird.

5. Verfahren zur Herstellung von N-(1-Cyclohexenylmethyl-2-pyrrolidinylmethyl)-6-ethylsulfonyl-chroman-8-carboxamid, dadurch gekennzeichnet, daß 6-Ethylsulfonyl-chroman-8-carbonsäure in Anwesenheit von Chlorameisensäureethylester mit 2,5-Dichlorpentylamin behandelt wird und anschließend das erhaltene N-(2,5-Dichlorpentyl)-6-ethylsulfonyl-chroman-8-carboxamid mit 1-Cyclohexenylmethylamin behandelt wird.

## Claims

### Claims for the following Contracting States: BE, CH, DE, FR, GB, IT, LI, LU, NL, SE

1. Dihydrobenzofuran- and chroman-carboxamide derivatives of formula (1) :

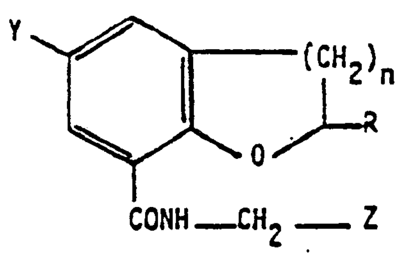

(I)

in which:

- R represents a hydrogen atom or a methyl group
- n is equal to 1 or 2,
- Z is a group of formula

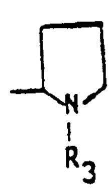

in which $R_3$ represents a cycloalkylalkyl or cycloalkenylalkyl group,

– Y is an chlorine aom or a cycloalkylmethylsufonyl group, and their pharmacologically acceptable acid addition salts and their optical isomers.

2. N-(1-cyclohexenylmethyl-2-pyrrolidinylmethyl)5-methylsulphamoy-2-methyl-2,3-dihydrobenzofuran-7-carboxamide.

3. N-(1-cyclohexenylmethyl-2-pyrrolidinylmethyl)-6-methylsulphamoyl-chroman-8-carboxamide.

4. According to Claim 1, N-(1-cyclohexenylmethyl-2-pyrrolidinylmethyl)5-cyclopropylmethylsulphonyl-2-methyl-2,3-dihydrobenzofuran-7-carboxamide.

5. According to Claim 1, N-(1-cyclopropylmethyl-2-pyrrolidinylmethyl)-6-cyclopropylmethylsulphonylchroman-8-carboxamide.

6. N-(1-cyclopropylmethyl-2-pyrrolidinylmethyl)-6-ethylsulphonylfroman-8-carboxamide.

7. N-(1-cyclopropylmethyl-2-pyrrolidinylmethyl)-5-ethylsulphonyl-2-methyl-2,3-dihydrobenzofuran-7-carboxamide.

8. N-(1-cyclohexenylmethyl-2-pyrrolidinylmethyl)-6-ethylsulphonylchroman-8-carboxamide.

9. According to Claim 1, N-(1-cyclohexenylmethyl-2-pyrrolidinylmethyl)-6-chlorochroman-8-carboxamide.

10. According to Claim 1, N-(1-cyclohexenylmethyl-2-pyrrolidinylmethyl)(5-chloro-2,3-dihydrobenzofuran-7-carboxamide.

11. According to Claim 1, N-(1-cyclohexenylmethyl-2-pyrrolidinylmethyl)5-chloro-2-methyl,-2,3-dihydrobenzofuran-7-carboxamide.

12. A method of preparing compounds of formula (1) according to Claim 1, comprising: treating an acid of formula (II):

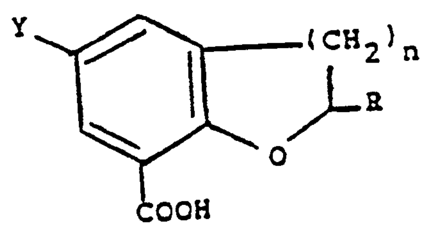

(II)

in which R, Y and n are defined as in Claim 1, or one of its reactive derivates, with an amine of formula (III)

$$H_2N-CH_2\text{--}Z \qquad (III)$$

in which Z is defined as in Claim 1, or alternatively, treating an acid of formula (II) or one of its reactive derivatives, with a dihaloalkylamine of formula (IV):

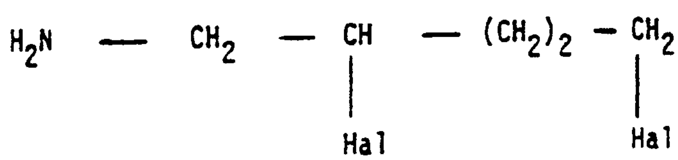

(IV)

in which Hal represents a halogen atom, then treating the compound obtained, of formula (V):

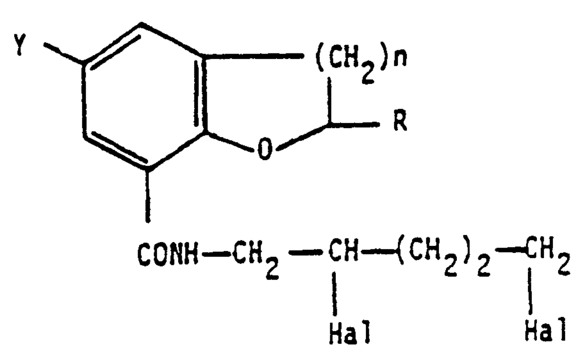

(V)

in which R, Y, n and Hal lare defined as above, with an amine of formula (VI)

$$H_2N\text{-}R_3 \qquad (VI)$$

in which $R_3$ is defined a sin Claim 1.

13. A method of preparing the ocmpound of Claim 2, comprising treating 5-methylsulphamoyl 2-methyl 2,3-dihydrobenzofuran 7-carbonyl chloride with 1-cyclohexenyl-methyl 2-aminomethylpyrrolidine.

14. A method of preparing the compound of Claim 3, comprising treating 6-methylsulphamoyl chroman 8-carboxylic acid with 1-cyclohexenylmethyl 2-aminomethyl pyrrolidine, in the presence of ethyl chloroformate.

15. A method of preparing the compounds of Claim 6 or 7, comprising treating 6-ethylsulphonyl chroman 8-carboxylic acid or 5-ethylsulphonyl 2-methyl 2,3-dihydrobenzofuran 7-carboxylic acid with 1-cyclopropylmehtyl 2-aminomethyl pyrrolidine, in the presence of ethyl chloroformate.

16. A method of preparing the compound of Claim 8, comprising treating 6-ethylsuphonyl chroman 8-carboxylic acid with 2,5-dichloropentylamine, in the presence of ethyl chloroformate, then treating the N-(2,5-dichloropentyl) 6-ethylsulphonyl chroman 8-carboxamide obained with 1-cyclohexenyl-methylamine.

17. The compound of any of Claims 1 to 11, used as medicaments, particularly as neuroleptics.

18. A pharmaceutical composition comprising a compound according to any of Claims 1 to 11, as the active principle, and a pharmaceutically acceptable excipient.

19. The compound of formula (V):

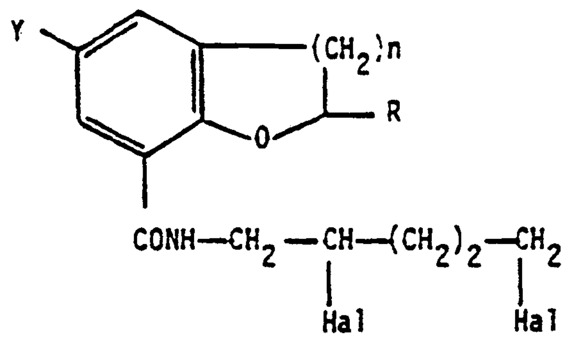

(V)

in which R, Y and n are defined as in Claim 1 and Hal is a halogen atom, as intermediate synthesising products used, in the method of Claim 12, for preparing the compound of formula (I).

20. N-(2,5-dichloropentyl) 6-ethylsulphonyl chroman 8-carboxamide as an intermediate synthesising product used in the method of Claim 16.

**Claims for the following Contracting States: AT, ES, GR**

1. A method of preparing dihydrobenzofuran- and chroman-carboxamide derivates of formula (1):

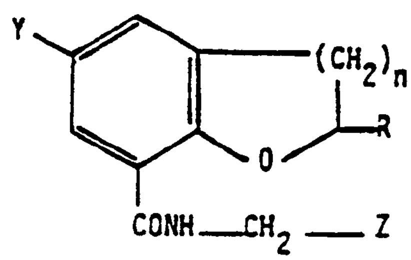

(I)

in which:

- R represents a hydrogen atom or a methyl group
- n is equal to 1 or 2,
- Z is a group of formula

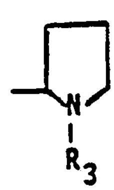

in which $R_3$ represents a cycloalkylalkyl or cycloalkenylalkyl group,
– Y is an chlorine atom or a cycloalkylmethylsufonyl group, and their pharmacologically acceptable aciud addition salts and their optical isomers, comprising: treating an acid of formula (II):

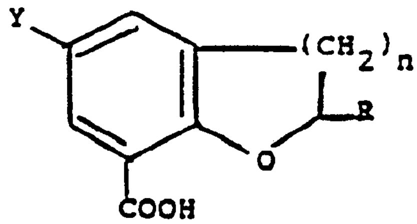

(II)

in which R, Y and n are defined as in formula (I), or one of its reactive derivatives, with an amine of formula (III)

$$H_2N - CH_2\text{--}Z \qquad (III)$$

in which Z is defined as in formula (I), or alternatively, treating an acid of formula (II) or one of its reactive derivatives, with a dihaloalkylamine of formula (IV):

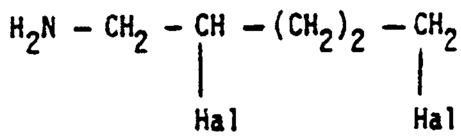

(IV)

in which Hal represents a halogen atom, then creating the compound obtained, of formula (V):

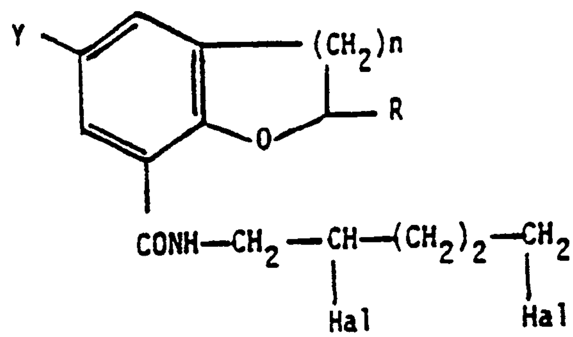

(V)

in which R, Y, n and Hal are defined as above, with an amine of formula (VI)

$$H_2N - R_3 \qquad (VI)$$

in which $R_3$ is defined as above.

2. A method of preparing N-(1-cyclohexenylmethyl-2-pyrrolidinylmethyl) 5-methylsulphamoyl-2-methyl-2,3-dihydrobenzofuran-7-carboxamide, comprising treating 5-methylsulphamoyl-2-methyl-2,3-dihydrobenzofuran-7-carbonyl chloride with 1-cyclohexenylmethyl-2-aminomethyl pyrrolidine.

3. A method of preparing N-(1-cyclohexenylmethyl-2-pyrrolidinylmethyl)-6-methylsulphamoyl-chroman-8-carboxamide, comprising treating 6-methylsulphamoyl-chroman-8-carboxylic acid with 1-cyclohexenylmethyl-2-aminomehtyl pyrrolidine, in the presence of ethychloroformate.

4. A method of preparing N-(1-cyclopropylmethyl-2-pyrrolidinylmethyl)-6-ethylsulphonylchroman-8-carboxamide or N(1-cyclopropylmethyl-2-pyrrolidinylmethyl)-5-ethylsulphonyl-2-methyl-2,3-dihydrobenzofuran-7-carboxamide, comprising treating 6-ethylsulphonylchroman-8-carboxylic acid or 5-ethylsulphonyl-2-methyl-2,3-dihydrobenzofuran-7-carboxylic acid with 1-cyclopropylmethyl-2-aminomethyl pyrrolidine, in the presence of ethylchloroformate.

5. A method of preparing N-(1-cyclohexenylmethyl-2-pyrrolidinylmethyl)-6-ethylsulphonylchroman-8-carboxamide, comprising treating 6-ethylsulphonylchroman-8-carboxylic acid with 2,5-dichloropentylamine, in the presence of ethyl chloroformate, then treating the N-(2,5-dichloropentyl-6-ethylsulphonylchroman-8-carboxamide obtained, with 1-cyclohexenylmethylamine.